# EUROPEAN PATENT APPLICATION

(11) **EP 0 976 776 A1**
(43) Date of publication of application: **02.02.2000**
(21) Application number: 99114874.3
(22) Date of filing: 28.07.1999
(51) Int. Cl.: C08J 3/05, A61L 27/00, A61K 7/48, C08L 89/06

(54) **Hypertonic aqueous dispersions of unfractioned collagen, a method and a device for the preparation of said dispersions**

(30) Priority: 31.07.1998 IT MI981797
(71) Applicant: EUROMED S.r.l., 40067 Rastignano (BO) (IT)
(72) Inventor: Pavani, Pier Giuseppe, 40067 Rastignano (BO) (IT)
(74) Representative: Minoja, Fabrizio, Dr.

(57) **Abstract**

The present invention relates to hypertonic aqueous dispersions of unfractioned collagen of bovine or equine origin, a method and a device for the preparation of said dispersions.

## Description

The present invention relates to hypertonic aqueous dispersions of unfractioned collagen, a method and a device for the preparation of said dispersions.

The dispersions of the invention are useful as injectable prosthesis.

Isotonic solutions of low molecular weight hydrolysed collagen have been used for some time as implants or prosthesis for correcting aesthetic imperfections such as scars, skin depressions of various nature, wrinkles, thin lips, and the like. Said collagen-based products are injected for inducing an increase in volume thus correcting profile or improving a functionality.

The uses of collagen have increased in time and nowadays collagen-based products are also used for the treatment of incontinence and vesicoureteral reflux in urology, and for the treatment of vocal cords in case of paresis, cordectomias or subtotal laryngectomias in otolaryngology.

The basic characteristics required for said products concern tolerability and persistence of the products in the injection site, as well as reduced costs of the treatment.

Collagen has up to now shown the best tolerability characteristics and the best grafting capability to the injection site; on the other hand, the cost and duration of the implant are insurmountable limits to the generalized diffusion of the treatment.

On the other hand, native collagen is an insoluble protein which is therefore subjected to a fractionation process in order to make it soluble in isotonic medium.

The sterile isotonic solutions of fractioned collagen suffers from a series of drawbacks which limit the uses thereof. In fact, the isotonic solutions, once injected into the anatomic site to be corrected, decrease in volume within a few hours (down to 1/4 of the starting volume), making therefore necessary to inject large volumes of the product. Furthermore, the isotonic sterile solutions of fractioned collagen should be stored at temperatures ranging from 2 to 9°C.

It has now been found that the above mentioned drawbacks can be overcome by using suitably prepared hypertonic dispersions of unfractioned collagen, in that collagen once injected, due to its hypertonicity, increases in volume by at least 3-4 times, giving rise to a more compact, elastic and lasting structure, while providing stable implants even when minimum amounts of product are injected.

The hypertonic dispersions of the invention preferably contain an about 5% w/v concentration of unfractioned collagen.

"Collagen" herein means any collagen from animal source, of average molecular weight higher than about 95,000-100,000 D, preferably collagen from bovine or equine tendons, more preferably collagen from equine tendons.

The hypertonic dispersions of the invention can further contain suitable excipients such as polyvinylpyrrolidone and the like, as well as the salts (sodium chloride, potassium and calcium) necessary to regulate hypertonicity.

The dispersions of the invention are prepared using a suitable homogenizing device which provides optimum results in terms of quality and stability of the dispersion.

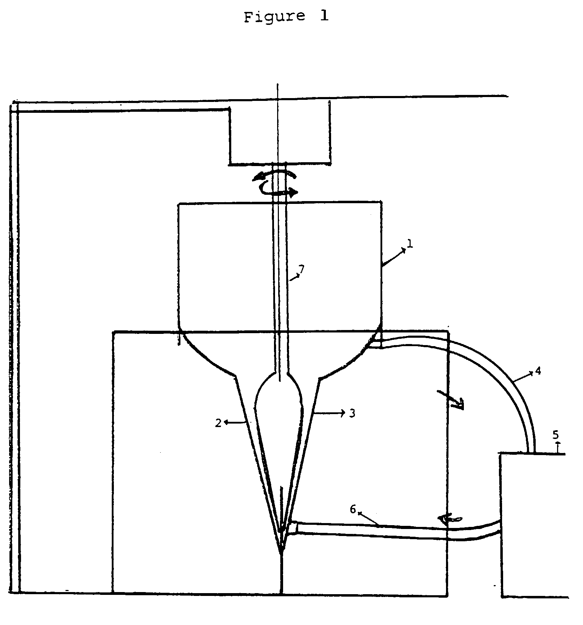

The device of the invention, the cross-section of which is schematically illustrated in the Figure, comprises a vessel (1) and a revolving element (2), placed inside the vessel (1) and coaxial to it, such as to define a space (3) into which is fed an aqueous dispersion of the proteins; the volume of this space (3) can vary continuously from a minimum to a maximum value through the reciprocal movement of the element (2) of the vessel (1); and means of feeding and circulating the aqueous dispersion (4-6).

The vessel (1) and the coaxial revolving element (2) are preferably conical in shape and can be made in any inert material such as stainless steel, glass, ceramic material, teflon and the like.

The revolving element (2) is connected to a motor capable of imparting a variable rotation speed which is typically between 10 and 200 rpm.

The distance between the walls of the vessel (1) and the revolving element (2) can be varied by raising or lowering the motor shaft (7), from a maximum value of about 5-7 mm to a minimum value of 0.1-0.3 mm.

The means of feeding and circulation typically comprise a peristaltic pump (5), an outlet pipe (4) and an inlet pipe (6).

The vessel (1) can be equipped with a thermostat.

The procedure for the preparation of the dispersions of the invention is carried out using the above described device according to the following operating steps.

A collagen suspension in physiological solution is added with acetic acid and placed in the vessel (1): in the first step, the element (2) is rotated at low speed (10-50 rpm) at a distance of 0.7-0.4 mm from the vessel.

The dispersion is continuously recycled by the peristaltic pump (5), the flow varying from about 0.2 to about 0.6 litres/minute.

The rotation speed is then gradually increased over a period of about 60-90 minutes, up to 100-150 rpm, progressively reducing the volume (3) and bringing nearer the element (2) down to a distance of about 0.2-0.3 mm from the walls of the vessel (1).

The recycle flow is increased up to about 3 litres/minute and left in this condition for a few hours (1-5 hours). The temperature is maintained at 40-45°C for the complete duration of the process.

The resulting fine dispersion can then be added with glutaraldehyde, in which case a further homogenization step is carried out in the device of the invention. Any glutaraldehyde traces can then be removed with conventional methods.

The sterile dispersions are then suitably formulated in a homogeneous gel which can finally be partitioned in syringes in amounts of 0.5-5 ml, as required. One of the advantages provided by the invention is that dispersions can be stored even at room temperature.

After administration, the dispersed collagen fibres firstly tend to hydrate, then they are subjected to over-extension thus taking up a three-dimensional space larger than that of normal implant collagens.

A subsequent phase follows, in which a biologically inert network forms which induces confluence of a large cell population, which will generate new filling material also making use of the anchoring protuberances of fibroblasts.

When the new filling material has reorganized the mass of injected collagen, long-term processes of remodelling, which are physiological but detrimental to the implant, will take place.

All these processes, from the hydration to the lysis of the implant, are rather slow, but the long-fibres structure of collagen resists up to 60 - 90 days before the network starts to break down (in hamster, which has high reactivity and very intense macrophagocytosis).

The device of the invention can be used for dispersing any protein or glycoprotein from animal or vegetable origin.

The following Examples illustrate the invention in greater detail.

### EXAMPLE 1

Freeze-dried collagen from equine tendons is suspended, in an amount of 4 g/100 ml, in a physiological solution containing 4% v/v acetic acid.

The non-homogeneous dispersion thus obtained is left to stand for about 30 minutes and is then placed in the device shown schematically in the Figure, with thermostatic control at 40°C. Rotation is started at 40 rpm, with a flow of 0.3 litres/minute and a distance of 4 mm between the vessel and the revolving piston. The rotation speed is then increased by 10 rpm every 10 minutes up to 110 rpm, and in bringing the piston nearer by 1 mm every 15 minutes down to 0.3 mm. The flow is then increased to 3 litres/minute and mixing is continued for 3 hours.

Glutaraldehyde is then added at a concentration of 3% w/v and stirring is continued at 110 rpm for 30 minutes with a distance of 0.2 mm to 0.3 mm between the piston and the vessel.

The whole is then centrifuged at 2000 rpm 10 times in physiological solution, each time discarding the supernatant whereas the precipitate is suspended in physiological solution adjusting pH to 6.5 ± 0.5.

Finally, two washings are carried out, suspending collagen in a hydrolyzed gelatin solution (Emagel), each time keeping the suspension stirred at a temperature of 40°C. These washings serve to completely remove any traces of glutaraldehyde attached to the molecules of hydrolyzed gelatin.

Two further washings are carried out with physiological solution and collagen is filtered off, then dried on the filter.

### EXAMPLE 2

### Injectable hypertonic dispersion

| **a) Composition** | **g/100 g** |
|---|---|
| (1) Collagen of Example 1 | g 5 |
| (2) Polyvinylpyrrolidone K 30 | g 3.5 |
| (3) Sodium chloride | g 0.85 |
| (4) Potassium chloride | g 0.038 |
| (5) Calcium chloride | g 0.07 |
| (6) Water for injectable preparations q.s. to | ml 100 |

### b) Preparation procedure

The desired amounts of polyvinylpyrrolidone K 30, sodium chloride, potassium chloride and calcium chloride are dissolved in water for injectable preparations.
- The solution is added with the necessary amount of collagen.
- The whole is stirred to completely disperse collagen thus obtaining a homogeneous gel.
- The gel is distributed in insulin syringes in amounts of ml 1.
- Each single syringe is packed in a heat sealed paper-PVC bag.

The single syringes packed are subjected to gamma-irradiation.

## Claims

1. Hypertonic aqueous dispersions of unfractioned collagen.

2. Dispersions as claimed in claim 1 in which collagen is from bovine or equine.

3. Dispersions as claimed in claim 1 or 2 in which the concentration of collagen is 5% w/v.

4. Pre-filled syringes containing a dispersion of claims 1-3.

5. A homogenizing device for proteins or glycoproteins of either animal or vegetable origin which includes a vessel (1) and a revolving element (2), placed inside the vessel (1) and coaxial to it, such that a space (3) is determined into which is fed an aqueous dispersion of proteins; where the volume of this space (3) can vary continuously from a minimum to a maximum value through the reciprocal movement of the element (2) and the vessel (1); and the means of feeding and circulating the aqueous dispersion (5).

6. A process for the preparation of the dispersions of claims 1-3 which comprises homogenizing an aqueous dispersion of unfractioned collagen in the device of claim 5.
